# EUROPÄISCHE PATENTSCHRIFT

(11) **EP 3 318 570 B1**
(45) Veröffentlichungstag und Bekanntmachung des Hinweises auf die Patenterteilung: **07.08.2019**
(21) Anmeldenummer: 16197717.8
(22) Anmeldetag: 08.11.2016
(51) Int. Cl.: C07F 15/00, C07C 45/50

(54) **PHOSPHORIGSÄURE-P,P'-[5,5',6,6'-TETRAMETHYL-3,3'-BIS(1-METHYLETHYL)[1,1'-BIPHENYL]-2,2'-DIYL] P,P,P',P'-TETRAKIS(2,4-DIMETHYLPHENYL)-ESTER IN DER HYDROFORMYLIERUNG**
PHOSPHORIC ACID-P,P'-[5,5',6,6'-TETRAMETHYL-3,3'-BIS(1-METHYLETHYL)[1,1'-BIPHENYL]-2,2'-DIYL] P,P,P',P'-TETRAKIS(2,4-DIMETHYLPHENYL)-ESTER IN HYDROFORMYLATION
ACIDE PHOSPHOREUX-P,P'-[5,5',6,6'-TÉTRAMÉTHYL-3,3'-BIS(1-ÉTHYLÉTHYL)[1,1'-BIPHÉNYL]-2,2'-DIYL] P,P,P',P'-TÉTRAKIS (2,4-DIMÉTHYLPHÉNYL)-ESTER DANS L'HYDROFORMYLATION

(43) Veröffentlichungstag der Anmeldung: 09.05.2018
(73) Patentinhaber: Evonik Degussa GmbH, 45128 Essen (DE)
(72) Erfinder: FRANKE, Robert, 45772 Marl (DE)

(56) Entgegenhaltungen:
- RU-C1- 2 584 952

## Beschreibung

Die Erfindung betrifft die Herstellung von Phosphorigsäure-P,P'-[5,5',6,6'-tetramethyl-3,3'-bis(1-methylethyl)[1,1'-biphenyl]-2,2'-diyl]P,P,P',P'-tetrakis(2,4-dimethylphenyl)-ester (Verbindung der Formel (**1**)), Mischungen umfassend diese Verbindung mit einem geringen Chlorgehalt sowie Metall-Komplexe dieser Verbindung. Des Weiteren betrifft diese Erfindung die Verwendung der Verbindung der Formel (**1**), wie auch derer Metall-Komplexe als katalytisch aktive Zusammensetzung in Hydroformylierungsreaktionen und die entsprechenden Verfahren selbst.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt. Als Katalysatoren in diesen Reaktionen werden häufig Verbindungen der Übergangsmetalle der VIII. Gruppe des Periodensystems der Elemente verwendet. Bekannte Liganden innerhalb dieser Katalysatoren sind beispielsweise Verbindungen aus den Klassen der Phosphine, Phosphite und Phosphonite mit jeweils dreiwertigen Phosphor P^{III}. Eine gute Übersicht über den Stand der Hydroformylierung von Olefinen findet sich in R. Franke, D. Selent, A. Börner, "Applied Hydroformylation", Chem. Rev., 2012, DOl:10.1021/cr3001803. Literaturbekannt ist die Synthese symmetrisch aufgebauter Bisphosphite, wie sie beispielsweise in US 4,769,498 offenbart werden, und deren Verwendung in katalytisch aktiven, übergangsmetallhaltigen Zusammensetzungen zur Hydroformylierung ungesättigter Verbindungen. RU 2584952 C1 offenbart Rhodium-Komplexe zur Hydroformylierung. In US 4,769,498, wie auch in US 5,723,641 werden bevorzugt symmetrisch aufgebaute Bisphosphite hergestellt und als Liganden zur Hydroformylierung verwendet. Die in der Hydroformylierung verwendeten symmetrisch aufgebauten Bisphosphitliganden werden bei tiefen Temperaturen hergestellt. Die Einhaltung dieser tiefen Temperaturen ist zwingend erforderlich, da höhere Temperaturen gemäß dieser US-Schriften zu Umlagerungen und letztlich zu unsymmetrisch aufgebauten Bisphosphiten führen würde.

Beschrieben ist zudem, dass symmetrische Liganden vorteilhafter sind als die entsprechenden unsymmetrischen Analoga.

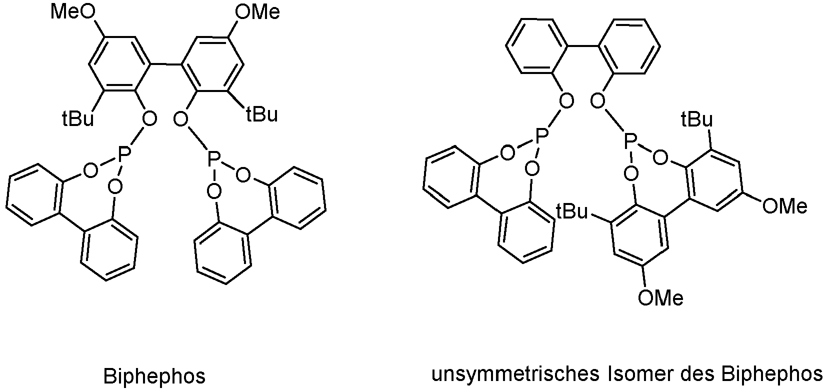

So sind beispielsweise in Rhodium-catalyzed Hydroformylation, ed. by P.W.N.M. van Leeuwen et C. Claver, Kluwer Academic Publishers 2006, AA Dordrecht, NL, Seite 45-46, Tabelle 2 die Hydroformylieriungsergebnisse der beiden oben abgebildeten Liganden unter vergleichbaren Bedingungen dargestellt. Dabei geht deutlich hervor, dass sich der symmetrische Biphephosligand (in der Literaturstelle Ligand 5a) durch eine deutlich höhere n/i-Selektivität und eine höhere Aktivität auszeichnet als sein unsymmetrisches Isomer (in der Literaturstelle Ligand 7). In der Hydroformylierungsreaktion von Propen weist der symmetrische Ligand eine n/i-Selektivität von 53 und eine Reaktionsrate von 402 auf, wohingegen der unsymmetrische Ligand lediglich eine n/i-Selektivität von 1.2 und eine Reaktionsrate von 280 aufweist. Wie durch diesen Vergleich gezeigt, weisen die unsymmetrisch aufgebauten Bisphosphite bei der Verwendung als Ligand in der übergangsmetallkatalysierten Hydroformylierung somit deutlich geringere Reaktivitäten und geringere n-Regioselektivitäten auf als die entsprechenden symmetrisch aufgebauten Verbindungen.

Der oben abgebildete symmetrische Biphephos-Ligand ist jedoch aufgrund seiner vielen Synthesestufen, insbesondere der Phenolkupplung sehr teuer in der Herstellung. Da der Ligandenpreis einen essentiellen Einfluss auf die Gesamtwirtschaftlichkeit eines Verfahrens hat, ist es wünschenswert, alternative Liganden mit vergleichbar guten Eigenschaften (Ausbeute und Regioselektivität) zu finden, die jedoch günstiger in ihrer Herstellung sind. Die technische Aufgabe der Erfindung ist die Bereitstellung eines symmetrischen Liganden, welcher in der Hydroformylierung von ungesättigten Verbindungen nicht die aus dem Stand der Technik zuvor aufgezeigten Nachteile, sondern eine oder mehrere der folgenden Eigenschaften aufweist:
1) eine gute Aktivität/Ausbeute,
2) eine hohe n-Regioseliktivität in Bezug auf die Hydroformylierung,
3) eine vergleichbar günstige Herstellung.

Die Aufgabe wird gelöst durch das hier vorgeschlagene Herstellungsverfahren der Verbindung der Formel (**1**) sowie deren Einsatz zur Katalyse in Hydroformylierungsverfahren. Die Verbindung (**1**) ist günstiger in der Herstellung als der oben genannte Biphephos-Ligand oder die mit Formel (**1**) in vergleichbarer Weise substituierte Verbindung der Formel (**3**) (siehe Vergleichsligand im Beispiel 2) und weist gleichzeitig eine sehr hohe n-Regioselektivität bei der Hydroformylierung auf und kann in hoher Ausbeute hergestellt werden.

Verbindung der Formel (**1**):

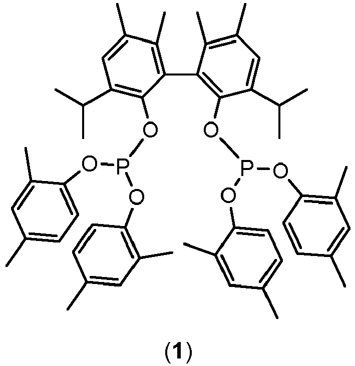

Gegenstand der vorliegenden Erfindung ist ein Komplex umfassend die Verbindung der Formel (**1**) sowie ein Metallatom ausgewählt aus Rh, Ru, Co und Ir. Die Verbindung der Formel (**1**) stellt hierbei vorzugsweise den Liganden in einem Ligand-Metall-Komplex dar.

Der Komplex liegt vorzugsweise in einer Form entsprechend Formel (**2**) vor, bei welcher das Metallatom M ausgewählt ist aus Rh, Ru, Co und Ir.

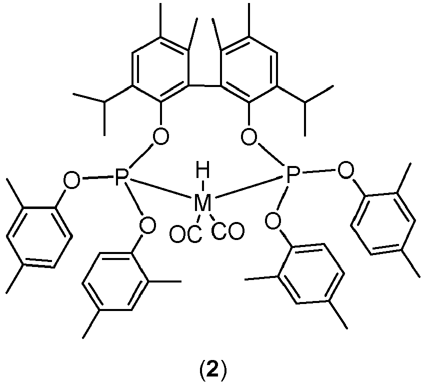

Mit besonderem Vorzug ist das Metallatom ein Rhodiumatom.

Gegenstand der vorliegenden Erfindung ist zudem ein Verfahren (Hydroformylierungsverfahren) umfassend die Verfahrensschritte:
a1) Vorlegen eines Olefins,
b1) Zugabe
   - eines Komplexes der Verbindung der Formel (1) mit Rh, Ru, Co oder Ir oder eines Komplexes der Formel (2) oder
   - einer Verbindung der Formel (1) und einer Substanz, welche ein Metallatom ausgewählt aus Rh, Ru, Co und Ir bereitstellt,
c1) Zuführen von H₂ und CO,
d1) Erwärmen des Reaktionsgemisches, wobei das Olefin zu einem Aldehyd umgesetzt wird.

Die Verfahrensschritte a1) bis c1) können hierbei in beliebiger Reihenfolge erfolgen.

Es kann in Bezug auf das Metallatom ein Überschuss an Verbindung der Formel (1) (Ligand) verwendet werden und nicht zwangsläufig jeder Ligand liegt gebunden in Form eines Ligand-Metall-Komplexes vor, sondern ist als "freier Ligand" im Reaktionsgemisch enthalten.

Die Reaktion wird bei üblichen Bedingungen durchgeführt.

Bevorzugt sind eine Temperatur von 80 °C bis 160 °C und ein Druck von 1 bis 300 bar.

Besonders bevorzugt sind eine Temperatur von 100 °C bis 160 °C und ein Druck von 15 bis 250 bar.

In einer bevorzugten Ausführungsform ist das Metall Rh.

Die Edukte für die Hydroformylierung gemäß dem Verfahren der Erfindung sind Olefine oder Gemische von Olefinen, insbesondere Monoolefine mit 2 bis 24, bevorzugt 3 bis 16, besonders bevorzugt 3 bis 12 Kohlenstoffatomen mit end- oder innenständigen C-C-Doppelbindungen, wie z.B. 1-Propen, 1-Buten, 2-Buten, 1- oder 2-Penten, 2-Methyl-1-buten, 2-Methyl-2-buten, 3-Methyl-1-buten, 1-, 2-, oder 3-Hexen, das bei der Dimerisierung von Propen anfallende C₆-Olefingemisch (Dipropen), Heptene, 2- oder 3-Methyl-1-hexene, Octene, 2-Methylheptene, 3-Methylheptene, 5-Methyl-2-hepten, 6-Methyl-2-hepten, 2-Ethyl-1-hexen, das bei der Dimerisierung von Butenen anfallende C₈-Olefingemisch (Di-n-buten, Di-iso-buten), Nonene, 2- oder 3-Methyloctene, das bei der Trimerisierung von Propen anfallende C₉-Olefingemisch (Tripropen), Decene, 2-Ethyl-1-octen, Dodecene, das bei der Tetramerisierung von Propen oder der Trimerisierung von Butenen anfallende C₁₂-Olefingemisch (Tetrapropen oder Tributen), Tetradecene, Hexadecene, das bei der Tetramerisierung von Butenen anfallende C₁₆-Olefingemisch (Tetrabuten) sowie durch Cooligomerisierung von Olefinen mit unterschiedlicher Anzahl von Kohlenstoffatomen (bevorzugt 2 bis 4) hergestellte Olefingemische.

Die eingesetzten Olefine können unsubstituiert oder substituiert sein. Geeignete Substituenten sind Carboxylgruppn, Aminogruppen, Carbonylgruppen, Halogene, insbesondere Chloride, Cyanogruppen und Sulfonsäuregruppen.

Bevorzugte Olefine sind Ethen, Propen und Buten, wobei unten den Begriff "Olefin" auch Olefingemische fallen.
Mittels des erfindungsgemäßen Verfahrens werden vorzugsweise (isomere) Propenale, Butanale, Pentanale, Nonanale oder Tridecanale, insbesondere Propenale oder Butanale hergestellt.

Mit dem erfindungsgemäßen Verfahren können unter Verwendung der erfindungsgemäßen Liganden α-Olefine, endständig verzweigte, innenständige und innenständig verzweigte Olefine hydroformyliert werden.

Besonders bevorzugt ist ein Verfahren umfassend die Verfahrensschritte:
a1) Vorlegen von Ethen, Propen oder Buten,
b1) Zugabe
   - eines Komplexes der Verbindung der Formel (1) mit Rh oder eines Komplexes der Formel (2) mit M = Rh oder
   - einer Verbindung der Formel (1) und einer Substanz, welche ein Rhodiumatom bereitstellt,
c1) Zuführen von H₂ und CO,
d1) Erwärmen des Reaktionsgemisches, wobei das Olefin zu einem Aldehyd umgesetzt wird,
wobei die Verfahrensschritte in der Reihenfolge a1, b1, c1, d1 durchlaufen werden.

Besonders bevorzugt ist ein Verfahren umfassend die Verfahrensschritte:
a1) Vorlegen von 1-Buten oder 2-Buten oder von Mischungen umfassend 1-Buten und/oder 2-Buten,
b1) Zugabe
   - eines Komplexes der Verbindung der Formel (1) mit Rh oder eines Komplexes der Formel (2) mit M = Rh oder
   - einer Verbindung der Formel (1) und einer Substanz, welche ein Rhodiumatom bereitstellt,
c1) Zuführen von H₂ und CO,
d1) Erwärmen des Reaktionsgemisches, wobei das Olefin zu einem Aldehyd umgesetzt wird,
wobei die Verfahrensschritte in der Reihenfolge a1, b1, c1, d1 durchlaufen werden.

Ein weiterer Gegenstand der vorliegenden Erfindung ist die Verwendung
- einer Verbindung der Formel (**1**) oder
- eines Komplexes der Verbindung der Formel (**1**) mit einem Metallkation ausgewählt aus Rh, Ru, Co oder Ir oder
- eines Komplexes der Formel (**2**)
zur Katalyse in einem Hydroformylierungsverfahren.

Vorzugsweise ist das Metallkation Rhodium.

Mit Vorzug wird
- die Verbindung der Formel (**1**) oder
- der Komplex der Verbindung der Formel (**1**) mit einem Metallkation ausgewählt aus Rh, Ru, Co oder Ir oder
- der Komplex der Formel (**2**)
zur Herstellung von Aldehyden mit 3 bis 20 Kohlenstoffatomen mittels eines Hydroformylierungsverfahrens, insbesondere zur Herstellung von Propenalen, Butanalen, Pentanalen, Nonanalen oder Tridecanalen mittels eines Hydroformylierungsverfahrens und mit besonderem Vorzug zur Herstellung von Propenalen oder Butanalen mittels eines Hydroformylierungsverfahrens verwendet. Hierbei können die resultierenden Aldehyde substituiert oder unsubstituiert sein.

Gegenstand der vorliegenden Erfindung ist des Weiteren ein Verfahren zur Herstellung einer Verbindung gemäß der Formel (1) 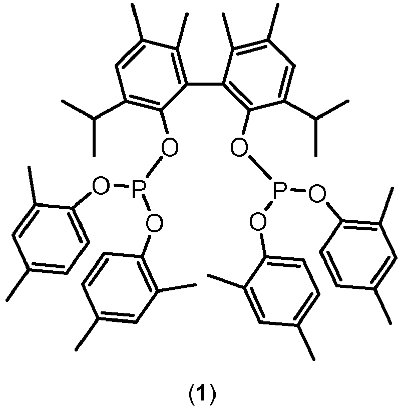 umfassend die Verfahrensschritte:
a2) Herstellung einer Zubereitung A2 umfassend Bis-(2,4-dimethylphenyl)-chlorophosphit in einem ersten Lösungsmittel,
b2) Herstellung einer Zubereitung B2 umfassend 3,3'-Diisopropyl-5,5',6,6'-tetramethyl-[1,1'-biphenyl]-2,2'-diol in einem zweiten Lösungsmittel,
c2) Mischen der Zubereitungen A2 und B2 bei einer Temperatur zwischen -50°C und Raumtemperatur.

Herstellungsverfahren der Edukte Bis-(2,4-dimethylphenyl)-chlorophosphit und 3,3'-Diisopropyl-5,5',6,6'-tetramethyl-[1,1'-biphenyl]-2,2'-diol sind in der Literatur beschrieben.

Unabhängig voneinander handelt es sich vorzugsweise bei den Zubereitungen A2 und B2 um Suspensionen oder Lösungen, insbesondere um Lösungen.

Mit Vorzug wird die Zubereitung B2 zu der Zubereitung A2 zudosiert. Hierzu wird vorzugsweise die Zubereitung A2 vor dem Zudosieren auf eine Temperatur zwischen -50 °C und Raumtemperatur, vorzugsweise auf eine Temperatur zwischen -40 °C und 10 °C, weiter bevorzugt auf eine Temperatur zwischen -35 °C und 0°C insbesondere auf eine Temperatur zwischen -25 °C und -10 °C gebracht.

Bis-(2,4-dimethylphenyl)-chlorophosphit und 3,3'-Diisopropyl-5,5',6,6'-tetramethyl-[1,1'-biphenyl]-2,2'-diol werden bevorzugt in einem Molverhältnis zwischen 4:1 und 1:1, besonders bevorzugt zwischen 3:1 und 1,3:1 und insbesondere zwischen 2,5:1 und 1,6:1 in dem oben beschriebenen Verfahren eingesetzt.

Als erstes und zweites Lösungsmittel können unabhängig voneinander aromatische Kohlenwasserstoffe wie insbesondere Toluol, Xylol, o-Xylol, Kresol; Ether, wie insbesondere Tetrahydrofuran (THF), Petrolether, Diethylether, Methyl-tert-butylether (MTBE); Nitrile wie insbesondere Acetonitril (ACN); Ethylacetat; Aceton; Alkohole wie insbesondere Methanol, Ethanol, Isopropanol, Butanol oder Mischungen dieser Lösungsmittel eingesetzt werden. Mit Vorzug kommen unpolare Lösungsmittel zum Einsatz.

In einer Ausführungsform des erfindunsgemäßen Verfahrens ist das erste und das zweite Lösungsmittel identisch und dabei vorzugsweise Toluol oder Xylol.

In einer anderen Ausführungsform unterscheiden sich erstes und zweites Lösungsmittel. Beispielsweise ist das erste Lösungsmittel ausgewählt aus Ethylacetat, Anisol, ortho-Xylol, Toluol, Aceton, Methanol, Ethanol, Propanol, iso-Propanol, Acetonitril und das zweite Lösungsmittel ausgewählt aus Ethylacetat, Anisol, ortho-Xylol, Toluol, Aceton, Methanol, Ethanol, Propanol, iso-Propanol, Acetonitril, Tetrahydrofuran, Diethylether, Glykol, C₅-C₁₀-Alkanen. Vorzugsweise ist das erste Lösungmittel Toluol oder Xylol und das zweite Lösungsmittel Acetonitril oder Methanol oder Ethanol.

Vorzugsweise enthält eine der Zubereitungen A2 oder B2 ein Amin ausgewählt aus Triethylamin, Dimethylaminobutan (DMAB), Pentylamin, Hexylamin, Dibutylamin, *N*-Methyl-2-pyrrolidon (NMP), Triethanolamin, Pyridin, Dimethylaminopyridin (DMAP). Vorzugsweise enthält nur die Zubereitung A2 Amin. Vorzugsweise wird Triethylamin oder Pyridin - vorzugsweise als Bestandteil der Zubereitung A2 - eingesetzt.

Berechnet auf das 3,3'-Diisopropyl-5,5',6,6'-tetramethyl-[1,1'-biphenyl]-2,2'-diol werden vorzugsweise 50 bis 500 Mol-%, bevorzugt 75 bis 400 Mol-%, besonders bevorzugt 100 bis 300 Mol-% Amin eingesetzt.

In einer ersten besonders bevorzugten Ausführungsform umfasst das Verfahren zur Herstellung einer Verbindung gemäß der Formel (1) 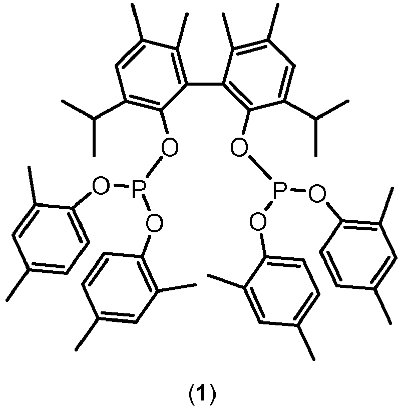 folgende Verfahrensschritte:
a2) Herstellung einer Suspension oder Lösung A2 umfassend Bis-(2,4-dimethylphenyl)-chlorophosphit, ein aromatisches Lösungsmittel, beispielsweise Xylol, Toluol oder Kresol und optional Amin, vorzugsweise in einer Menge von 100 - 300 Mol-% (berechnet auf das 3,3'-Diisopropyl-5,5',6,6'-tetramethyl-[1,1'-biphenyl]-2,2'-diol in B2),
b2) Herstellung einer Suspension oder Lösung B2 umfassend 3,3'-Diisopropyl-5,5',6,6'-tetramethyl-[1,1'-biphenyl]-2,2'-diol und ein aromatisches Lösungsmittel, beispielsweise Xylol, Toluol oder Kresol,
c2) schrittweises, vorzugsweise kontinuierliches Zudosieren von B2 zu A2, wobei
   - das Molverhältnis von Bis-(2,4-dimethylphenyl)-chlorophosphit zu 3,3'-Diisopropyl-5,5',6,6'-tetramethyl-[1,1'-biphenyl]-2,2'-diol zwischen 3:1 und 1,3:1, insbesondere zwischen 2,5:1 und 1,6:1 liegt und
   - die Suspension oder Lösung A2 vor dem Zudosieren von B2 auf eine Temperatur von -50°C und -0 °C, insbesondere auf eine Temperatur von -30°C und 0 °C gebracht wird.

Vorzugsweise wird hierbei in den Schritten a2 und b2 dasselbe Lösungsmittel eingesetzt.

In einer weiteren besonders bevorzugten Ausführungsform umfasst das Verfahren zur Herstellung einer Verbindung gemäß der Formel (1) 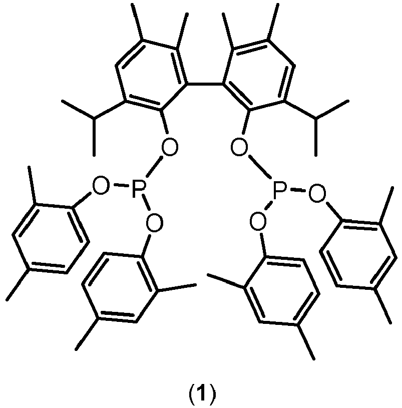 folgende Verfahrensschritte:
a2) Herstellung einer Suspension oder Lösung A2 umfassend Bis-(2,4-dimethylphenyl)-chlorophosphit, Toluol und optional Amin, vorzugsweise in einer Menge von 100 - 300 Mol-% (berechnet auf das 3,3'-Diisopropyl-5,5',6,6'-tetramethyl-[1,1'-biphenyl]-2,2'-diol in B2),
b2) Herstellung einer Suspension oder Lösung B2 umfassend 3,3'-Diisopropyl-5,5',6,6'-tetramethyl-[1,1'-biphenyl]-2,2'-diol und Toluol,
c2) schrittweises, vorzugsweise kontinuierliches Zudosieren von B2 zu A2,
   wobei
   - das Molverhältnis von Bis-(2,4-dimethylphenyl)-chlorophosphit zu 3,3'-Diisopropyl-5,5',6,6'-tetramethyl-[1,1'-biphenyl]-2,2'-diol zwischen 3:1 und 1,3:1, insbesondere zwischen 2,5:1 und 1,6:1 liegt und
   - die Suspension oder Lösung A2 vor dem Zudosieren von B2 auf eine Temperatur von -30°C und 0 °C gebracht wird.

Mit Vorzug wird in dem erfindungsgemäßen Verfahren zur Herstellung der Verbindung der Formel (**1**) nach den beschriebenen Verfahrensschritten ein Verfahrensschritt durchgeführt, welcher der Reduzierung des Gesamtchlorgehaltes dient. Die Reduzierung des Gesamtchlorgehaltes umfasst bevorzugt die folgenden Schritte:
i. Lösen der Verbindung der Formel (**1**) in einem ersten Lösungsmittel oder ersten Lösungsmittelgemisch bei einer Temperatur T zwischen Raumtemperatur und dem Siedepunkt der Lösung, vorzugsweise bei mindestens 40°C, bevorzugt bei mindestens 60 °C und insbesondere bei einer Temperatur, die 2 bis 15 °C unterhalb des Siedepunktes der Lösung liegt;
ii. Rühren der Lösung aus Schritt i);
iii. Abkühlen der Lösung aus Schritt ii) um mindestens 10 °C, vorzugsweise um 20 °C oder mindestens 30°C unter Bildung einer Suspension oder Emulsion;
iv. Halten, vorzugsweise Rühren der Suspension oder Emulsion aus Schritt iii) für 2 bis 50 Stunden;
v. Seperieren der Phasen der Suspension oder Emulsion aus Schritt iv).

Sollte sich hierbei im Schritt i) keine klare Lösung bilden, werden unlösliche Bestandteile vorzugsweise abgetrennt und verworfen.

Es resultiert vorzugsweise ein Produkt umfassend die Verbindung der Formel (1) mit einem Chlorwert von 10 ppm bis 10 000 ppm, vorzugsweise von 20 ppm bis 5000 ppm, weiter bevorzugt von 30 bis 1000 ppm und insbesondere unterhalb von 500 oder sogar unterhalb von 300 ppm.

Dementsprechend ist es besonders bevorzugt, dass
- die Verbindung der Formel (**1**) oder
- der Komplex der Verbindung der Formel (**1**) mit einem Metallkation ausgewählt aus Rh, Ru, Co oder Ir oder
- der Komplex der Formel (**2**)
   einen Chlorwert in dem oben angegebenen Bereich aufweist und/oder
- die Verbindung der Formel (**1**) oder
- der Komplex der Verbindung der Formel (**1**) mit einem Metallkation ausgewählt aus Rh, Ru, Co oder Ir oder
- der Komplex der Formel (**2**)
mit einem Chlorwert in dem oben angegebenen Bereich in dem erfindungsgemäßen Hydroformylierungsverfahren eingesetzt wird und/oder erfindungsgemäß zur Katalyse in einem Hydroformylierungsverfahren verwendet wird.

Weiterer Gegenstand der vorliegenen Erfindung ist dementsprechend eine Mischung umfassend die Verbindung der Formel (**1**), dadurch gekennzeichnet, dass diese Mischung einen Chlorwert - bestimmt nach Wickbold - unterhalb von 10.000 ppm, insbesondere unterhalb von 1.000 ppm aufweist.

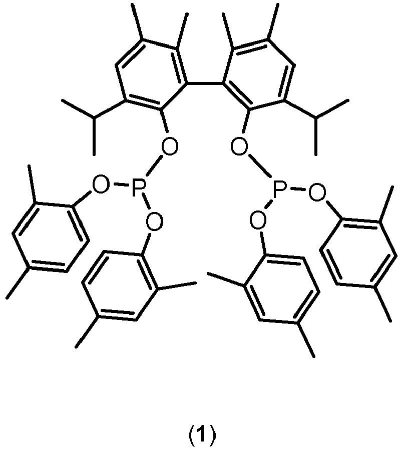

Mit Vorzug liegt der Chlorwert dabei unterhalb von 500 ppm, bevorzugt unterhalb von 400 ppm, vorzugsweise unterhalb von 300 ppm und weiter bevorzugt unterhalb von 200 ppm, wobei der Chlorwert sich auf das Gewicht bezieht.

Diese Ausgestaltung der beschriebenen Erfindung resultiert daraus, dass Verunreinigungen in Form von Chlorverbindungen Schwierigkeiten bereiten: Gelangen die chlorhaltigen Verunreinigungen zusammen mit der als Ligand verwendeten Organophosphorverbindung in einen stählernen Druckreaktor, so ist dieser durch das Chlorid erhöhter Korrosion ausgesetzt. Dies gilt ganz besonders für kontinuierlich betriebene Prozesse, bei denen die Organophosphorverbindungen im Laufe der Reaktion nachdosiert werden. Dies ist beispielsweise bei der Verwendung der Organophosphorverbindung als Ligand in der großtechnischen Hydroformylierung der Fall. Durch die Nachdosierung kommt es zwangsläufig auch zu einer Akkumulation der Nebenkomponenten im Reaktor. Dies ist insbesondere dann kritisch, wenn Chlorid eine der Nebenkomponten darstellt, da Chlorid selbst rostfreie Stähle angreift. In Anwesenheit von Chloridionen droht insbesondere eine Spannungsrisskorrosion, welche im günstigeren Fall zu einer vorzeitigen Abstellung des Prozesses und zur Überholung des Reaktors führt, im ungünstigsten Fall aber auch ein Bersten des Reaktors zur Folge haben kann. Es ist daher von eminenter Bedeutung, ein Einschleppen von chlorhaltigen Verbindungen über das organophosphorhaltige Katalysatorsystem zu unterbinden.

Der Chloridgehalt lässt sich analytisch einfach bestimmen; zum Beispiel durch wässrige Titration. Weitreichender ist die Bestimmung des Gesamtchlorgehalts, der neben den Chloriden auch anderweitig gebundenes Chlor umfasst. Ein Abstellen auf den Gesamtchlorgehalt ist auch insoweit sachdienlich, als nicht ausgeschlossen werden kann, dass auch anderweitig gebundenes Chlor den Reaktor zu schädigen vermag. Bei der Bemessung der Grenzwerte für Gesamtchlor bleibt aber der Chloridanteil maßgeblich. Ein geeignetes Verfahren zur Bestimmung des Gesamtchlorgehalts ist die Verbrennung nach Wickbold mit Probenvorbereitung nach DIN 51408 und Messung per Ionenchromatographie nach DIN EN ISO 10304. Die im Rahmen dieser Erfindung beschriebenen Chlorwerte wurden ermittelt nach Wickbold oder mittels Röntgenfluoreszenzanalyse (RFA).

Im Folgenden soll die Erfindung anhand von Ausführungsbeispielen näher erläutert werden.

### Beispiele:

Alle nachfolgenden Präparationen wurden mit Standard-Schlenk-Technik unter Schutzgas durchgeführt. Die Lösungsmittel wurden vor Gebrauch über geeigneten Trocknungsmitteln getrocknet (Purification of Laboratory Chemicals, W. L. F. Armarego (Autor), Christina Chai (Autor), Butterworth Heinemann (Elsevier), 6. Auflage, Oxford 2009). Alle präparativen Arbeiten erfolgten in ausgeheizten Gefäßen. Die Charakterisierung der Produkte erfolgte mittels NMR-Spektroskopie. Chemische Verschiebungen (δ) werden in ppm angegeben. Die Referenzierung der ³¹P-NMR-Signale erfolgte gemäß: SR_{31P} = SR_{1H} ^{∗} (BF_{31P} / BF_{1H}) = SR_{1H} ^{∗} 0,4048. (Robin K. Harris, Edwin D. Becker, Sonia M. Cabral de Menezes, Robin Goodfellow, and Pierre Granger, Pure Appl. Chem., 2001, 73, 1795-1818; Robin K. Harris, Edwin D. Becker, Sonia M. Cabral de Menezes, Pierre Granger, Roy E. Hoffman and Kurt W. Zilm, Pure Appl. Chem., 2008, 80, 59-84). Die Aufnahme von Kernresonanzspektren erfolgte an Bruker Avance 300 bzw. Bruker Avance 400.

### Beispiel 1: Umsetzung von Bis-(2,4-dimethylphenyl)-chlorophosphit mit 3,3'-Diisopropyl-5,5',6,6'-tetramethyl-[1,1'-biphenyl]-2,2'-diol

8 g (0,020 mol) Bis-(2,4-dimethylphenyl)-chlorophosphit wurden bei Raumtemperatur in 50 ml Toluol unter Zusatz von 3,9 ml (2,85 g, 0,028 mol) Triethylamin gelöst und auf -20 °C temperiert. Zu dieser Lösung wurde eine Lösung von 3,1 g (0,009 mol) 3,3'-Diisopropyl-5,5',6,6'-tetramethyl-[1,1'-biphenyl]-2,2'-diol in 50 ml Toluol innerhalb von 12 Minuten, unter Rühren, kontinuierlich zugegeben. Nach vollständiger Zugabe wurde unter Rühren auf Raumtemperatur erwärmt. Anschließend wurde das bei der Reaktion entstandene Hydrochlorid mittels Quarzglasfilter (Fritte) abgetrennt und das Filtrat mittels Ölpumpenvakuum (10⁻³ mbar) bei Raumtemperatur bis zur Trockene eingeengt. Der resultierende Feststoff wurde in 50 ml Acetonitril 1 h kräftig gerührt. Nach der Trennung der Phasen wurde die obere Phase abdekantiert und die untere Phase im Ölpumpenvakuum (10⁻³ mbar) bei Raumtemperatur getrocknet. Gemäß ¹H-NMR, ³¹P-NMR und ³¹P-¹H-HMBC-NMR (gelöst in Toluol-ds) enthält der hochviskose Rückstand 73,3 % Verbindung der Formel (1). Chlorwert nach Wickbold: 58 ppm.

### Beispiel 2: Hydroformylierung

In einem 100 ml-Autoklaven der Fa. Parr Instruments wurde bei 120 °C und 20 bar Synthesegasdruck (CO/H₂ = 1:1 (Vol-%)) 5,6 g cis-2-Buten hydroformyliert. Als Precursor wurde Rh(acac)(CO)₂ in 48,8 g Toluol vorgelegt. Der Ligand wurde im molaren Überschuss von 4:1 relativ zum Rhodium verwendet. Als Ligand wurden 0,0779 g Ligand in der Katalysatoransatzlösung eingesetzt. Als Stabilisator wurde Tinuvin 770DF im molaren Verhältnis ca. 1:1 zum Liganden eingesetzt. Zusätzlich wurde ein GC-Standard zugefügt. Ca. 6 g Edukt wurden nach Erreichen der vorgesehenen Reaktionstemperatur zudosiert.

Während der Reaktion wurde der Druck über eine Synthesegasregelung mit Massedurchflussmesser konstant gehalten. Die Rührerdrehzahl betrug 1200 min⁻¹. Proben wurden aus der Reaktionsmischung nach 12 Stunden gezogen.

Weiterhin wurde die Verbindung (3) unter entsprechenden Bedingungen getestet. Verbindung (3) wurde gemäß EP 2 907 819 A1 hergestellt.

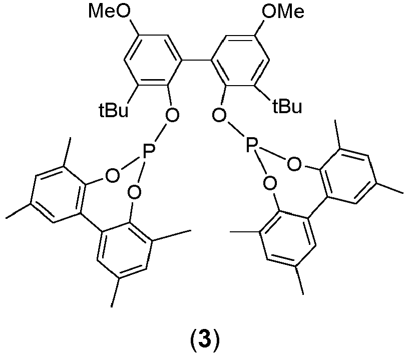

In Tabelle 1 sind die Hydroformylierungsergebnisse von cis-2-Buten bei 20 bar Synthesegasdruck dargestellt.

**Tabelle 1:**

| Ligand = Verbindung (X) X = | Ausbeute Aldehyd in [%] | Regioselektivität n-Pentanal in % |
|---|---|---|
| **1*** | 79 | 98 |
| **3** | 66 | 90 |

| | | |
|---|---|---|
| * erfindungsgemäße Verbindung der Formel (1) | | |

### Definition der Selektivität:

Bei der Hydroformylierung gibt es die n/iso-Selektivitäte (n/iso = das Verhältnis von linearem Aldehyd (= n) zu verzweigtem (= iso) Aldehyd)). Hierbei bedeutet die Regioselektivität n-Pentanal, dass diese Menge an linearem Produkt gebildet wurde. Die restlichen Prozente entsprechen dann dem verzweigten Isomer. Die Selektivitätsrate wurde mittels Flächenvergleich im GC bestimmt.

Die Ergebnisse zeigen, dass die Verbindung (**1**) bei der Hydroformylierung sowohl eine höhere Ausbeute ermöglicht, also reaktiver ist, als sich auch durch eine höhere Regioselektivität hinsichtlich des n/iso-Verhältnisses auszeichnet als die Verbindung (**3**).

## Patentansprüche

1. Komplex umfassend eine Verbindung gemäß der Formel (1) 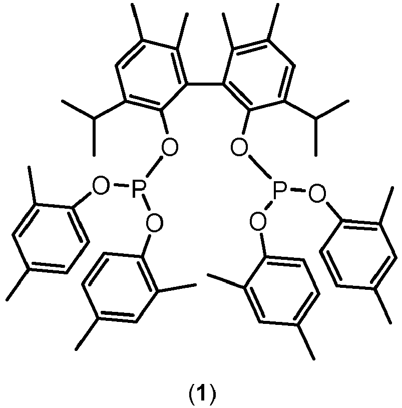 sowie ein Metallatom ausgewählt aus: Rh, Ru, Co und Ir.

2. Komplex gemäß Anspruch 1, wiedergegeben durch Formel (2) mit M = Metallatom ausgewählt aus Rh, Ru, Co und Ir.

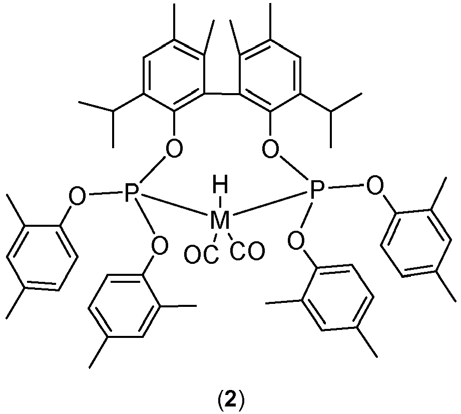

3. Komplex gemäß einem der Ansprüche 1 oder 2, **dadurch gekennzeichnet, dass** das Metallatom Rh ist.

4. Verfahren umfassend die Verfahrensschritte:
a1) Vorlegen eines Olefins,
b1) Zugabe
- eines Komplexes gemäß einem der Ansprüche 1 oder 2 oder
- einer Verbindung gemäß der Formel (**1**) aus Anspruch 1 und einer Substanz, welche ein Metallatom ausgewählt aus: Rh, Ru, Co und Ir bereitstellt,
c1) Zuführen von H₂ und CO,
d1) Erwärmen des Reaktionsgemisches, wobei das Olefin zu einem Aldehyd umgesetzt wird.

5. Verfahren gemäß Anspruch 4, **dadurch gekennzeichnet, dass** das Metallatom Rh ist.

6. Verfahren gemäß einem der Ansprüche 4 oder 5, **dadurch gekennzeichnet, dass** im Schritt a1) ein Olefin mit 2 bis 24 Kohlenstoffatomen, vorzugsweise Ethen, Propen oder Buten, insbesondere 1-Buten oder/oder 2-Buten vorgelegt wird.

7. Verwendung
- einer Verbindung gemäß Formel (**1**) aus Anspruch 1 oder
- eines Komplexes gemäß einem der Ansprüche 1 bis 3
zur Katalyse in einem Hydroformylierungsverfahren.

8. Verwendung gemäß Anspruch 7, **dadurch gekennzeichnet, dass** mittels des Hydroformylierungsverfahrens substituierte oder unsubstituierte Aldehyde mit 3 bis 20 Kohlenstoffatomen, vorzugsweise substituierte oder unsubstituierte Propenale, Butanale, Pentanale, Nonanale oder Tridecanalen, insbesondere substituierte oder unsubstituierte Propenale, Butanale oder Pentanale hergestellt werden.

9. Verfahren zur Herstellung einer Verbindung gemäß der Formel (**1**) 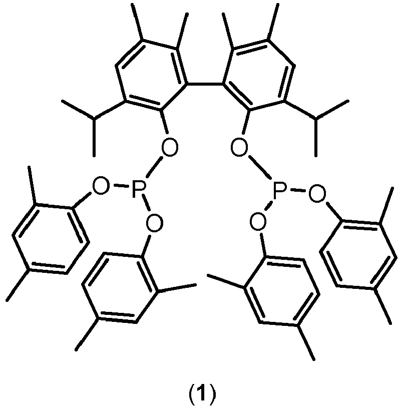 umfassend die Verfahrensschritte:
a2) Herstellung einer Zubereitung A2 umfassend Bis-(2,4-dimethylphenyl)-chlorophosphit in einem ersten Lösungsmittel;
b2) Herstellung einer Zubereitung B2 umfassend 3,3'-Diisopropyl-5,5',6,6'-tetramethyl-[1,1'-biphenyl]-2,2'-diol in einem zweiten Lösungsmittel;
c2) Mischen der Zubereitungen A2 und B2 bei einer Temperatur zwischen -50°C und Raumtemperatur.

10. Verfahren gemäß Anspruch 9, **dadurch gekennzeichnet, dass** die Zubereitung B2 zur Zubereitung A2 bei -50 °C bis 0 °C zudosiert wird.

11. Mischung umfassend die Verbindung der Formel (**1**), **dadurch gekennzeichnet, dass** diese Mischung einen Chlorwert - bestimmt nach Wickbold - unterhalb von 10.000 ppm, insbesondere unterhalb von 1.000 ppm aufweist, wobei sich der Chlorwert auf das Gewicht bezieht.

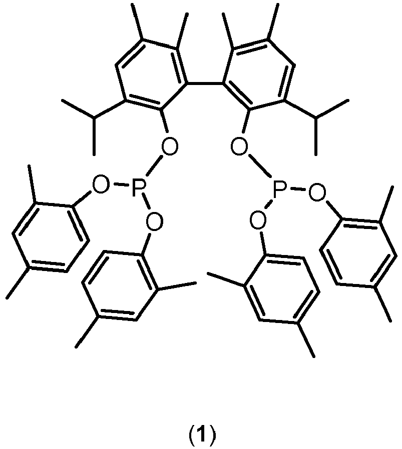

## Claims

1. Complex comprising a compound of the formula (**1**) 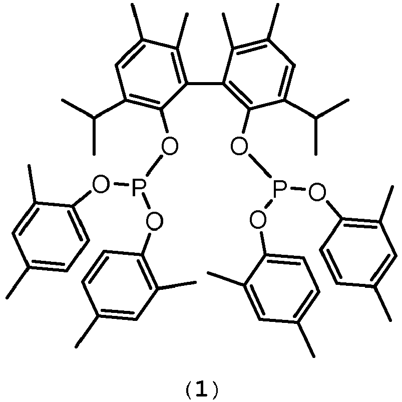 and a metal atom selected from: Rh, Ru, Co and Ir.

2. Complex according to Claim 1, represented by formula **(2)** with M = metal atom selected from Rh, Ru, Co and Ir.

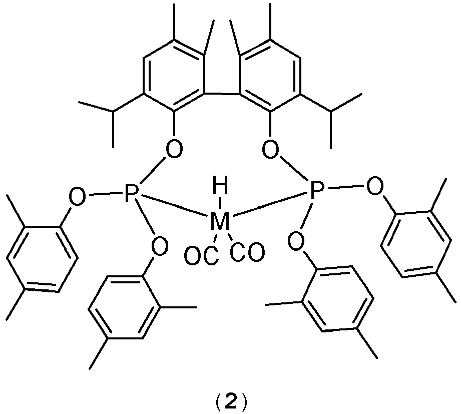

3. Complex according to either of Claims 1 and 2, **characterized in that** the metal atom is Rh.

4. Process comprising the process steps of:
a1) initially charging an olefin,
b1) adding
- a complex according to either of Claims 1 and 2 or
- a compound of the formula (**1**) from Claim 1 and a substance that provides a metal atom selected from: Rh, Ru, Co and Ir,
c1) feeding in H₂ and CO,
d1) heating the reaction mixture, with conversion of the olefin to an aldehyde.

5. Process according to Claim 4, **characterized in that** the metal atom is Rh.

6. Process according to either of Claims 4 and 5, **characterized in that**, in step a1), an olefin having 2 to 24 carbon atoms, preferably ethene, propene or butene, especially 1-butene and/or 2-butene, is initially charged.

7. Use of
- a compound of formula (1) from Claim 1 or
- a complex according to any of Claims 1 to 3 for catalysis in a hydroformylation process.

8. Use according to Claim 7, **characterized in that**, by means of the hydroformylation process, substituted or unsubstituted aldehydes having 3 to 20 carbon atoms, preferably substituted or unsubstituted propenals, butanals, pentanals, nonanals or tridecanals, especially substituted or unsubstituted propenals, butanals or pentanals, are prepared.

9. Process for preparing a compound of the formula (1): 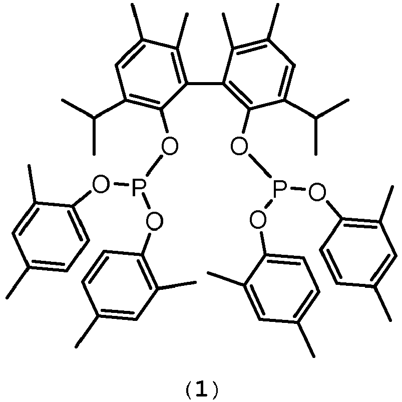 comprising the following process steps:
a2) preparing a formulation A2 comprising bis(2,4-dimethylphenyl) chlorophosphite in a first solvent;
b2) preparing a formulation B2 comprising 3,3'-diisopropyl-5,5',6,6'-tetramethyl-[1,1'-biphenyl]-2,2'-diol in a second solvent;
c2) mixing formulations A2 and B2 at a temperature between -50°C and room temperature.

10. Process according to Claim 9, **characterized in that** formulation B2 is metered into formulation A2 at - 50°C to 0°C.

11. Mixture comprising the compound of the formula (1), **characterized in that** this mixture has a chlorine value - determined according to Wickbold - below 10 000 ppm, especially below 1000 ppm, where the chlorine value is based on the weight.

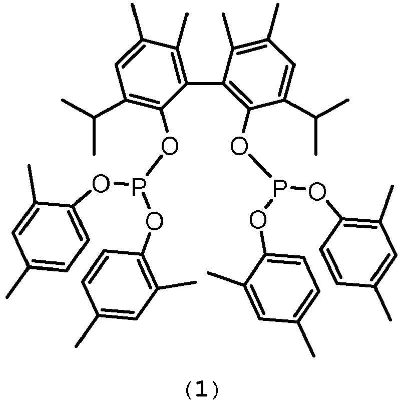

## Revendications

1. Complexe comprenant un composé selon la formule (1) 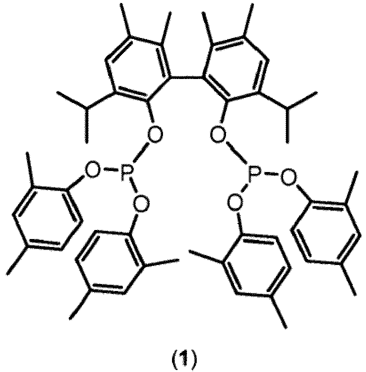 ainsi qu'un atome métallique choisi parmi : Rh, Ru, Co et Ir.

2. Complexe selon la revendication 1, représenté par la formule (2) avec M = atome métallique choisi parmi Rh, Ru, Co et Ir

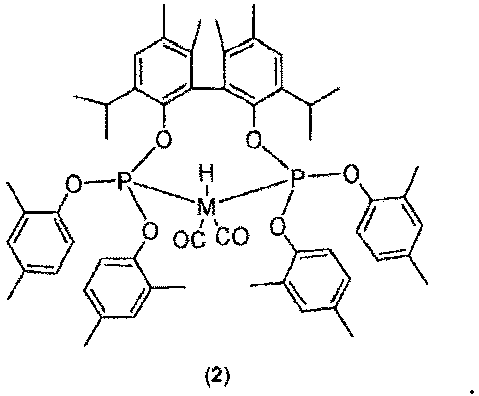

3. Complexe selon l'une quelconque des revendications 1 ou 2, **caractérisé en ce que** l'atome métallique est Rh.

4. Procédé comprenant les étapes de procédé suivantes :
a1) le chargement d'une oléfine,
b1) l'ajout
- d'un complexe selon l'une quelconque des revendications 1 ou 2, ou
- d'un composé de formule (1) selon la revendication 1 et d'une substance qui met à disposition un atome métallique choisi parmi : Rh, Ru, Co et Ir,
c1) l'introduction d'H₂ et de CO,
d1) le chauffage du mélange réactionnel, l'oléfine étant transformée en un aldéhyde.

5. Procédé selon la revendication 4, **caractérisé en ce que** l'atome métallique est Rh.

6. Procédé selon l'une quelconque des revendications 4 ou 5, **caractérisé en ce qu'**à l'étape a1), une oléfine contenant 2 à 24 atomes de carbone, de préférence l'éthène, le propène ou le butène, notamment le 1-butène et/ou le 2-butène, est chargée.

7. Utilisation
- d'un composé de formule (1) selon la revendication 1 ou
- d'un complexe selon l'une quelconque des revendications 1 à 3
pour la catalyse dans un procédé d'hydroformylation.

8. Utilisation selon la revendication 7, **caractérisée en ce que** des aldéhydes substitués ou non substitués de 3 à 20 atomes de carbone, de préférence des propénals, butanals, pentanals, nonanals ou tridécanals substitués ou non substitués, notamment des propénals, butanals ou pentanals substitués ou non substitués, sont fabriqués par le procédé d'hydroformylation.

9. Procédé de fabrication d'un composé selon la formule (1) 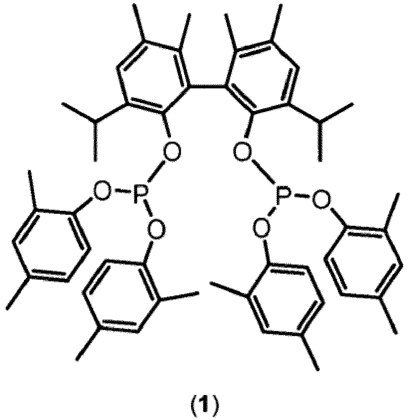 comprenant les étapes de procédé suivantes :
a2) la fabrication d'une préparation A2 comprenant du bis-(2,4-diméthylphényl)-chlorophosphite dans un premier solvant ;
b2) la fabrication d'une préparation B2 comprenant du 3,3'-diisopropyl-5,5' ,6,6'-tétraméthyl-[1,1'-biphényl]-2,2'-diol dans un deuxième solvant ;
c2) le mélange des préparations A2 et B2 à une température comprise entre -50 °C et la température ambiante.

10. Procédé selon la revendication 9, **caractérisé en ce que** la préparation B2 est ajoutée à la préparation A2 à une température de -50 °C à 0 °C.

11. Mélange comprenant le composé de formule (1), **caractérisé en ce que** ce mélange présente une valeur de chlore, déterminée selon Wickbold, inférieure à 10 000 ppm, notamment inférieure à 1 000 ppm, la valeur de chlore se rapportant au poids
